# EUROPEAN PATENT APPLICATION

(11) **EP 3 790 252 A1**
(43) Date of publication of application: **10.03.2021**
(21) Application number: 20194964.1
(22) Date of filing: 08.09.2020
(51) Int. Cl.: H04L 29/06, G16H 10/65, H04L 9/08, H04L 9/32

(54) **SMART CARD PASSWORD MANAGEMENT SYSTEMS AND METHOD**

(30) Priority: 09.09.2019 US 201962897623 P; 24.06.2020 US 202016910241
(71) Applicant: Medtronic, Inc., Minneapolis, MN 55432 (US)
(72) Inventor: Parari, Vijaya Kumar, Fridley, MN Minnesota 55432 (US); Poindexter, Rebecca, Minneapolis, MN Minnesota 55413 (US); Ghosh Roy, Arindam, 560049 Bangalore KA (IN)
(74) Representative: Maschio, Antonio

(57) **Abstract**

The invention relates to devices, systems, and methods for controlling smart card authentication for a system. The systems and methods can include a smart card reader for gaining access to a smart card by sending a first password to access the smart card wherein the smart card reader can read a unique ID of the smart card and use an algorithm to generate a second password and store the generated second password directly in the smart card's file directory. The medical systems can include any medical device or machine requiring the transfer of any data such as personal health information (PHI) and therapy parameter data to be used by medical devices in the system. The methods, algorithms, and processes can also be used as a standard for any number of devices and systems for accessing a particular device.

## Description

### CROSS-REFERENCE TO RELATED APPLICATION

This application claims the benefit of and priority to U.S. Provisional Patent Application No. 62/897,623 filed September 9, 2019, the entire disclosure of which is incorporated by reference herein.

### FIELD

The disclosure relates to devices, systems, methods, and smartcards for controlling smart card authentication for a medical system. The medical systems can include any medical device or machine requiring the transfer of any data such as personal health information (PHI) and therapy parameter data to be used by medical devices in the systems. The methods, algorithms, and processes described herein can serve as a basis for a standard to be used by any number of devices and systems for accessing a particular device.

### BACKGROUND

New distributed healthcare models including value-based care systems and precision medical devices such as home-based and portable dialysis units can require the transfer of personal health information (PHI) and other data to function properly. The Health Insurance Portability and Accountability Act (HIPAA) Security Rule enacted in 1997 in the United States and other similar laws throughout the world establish standards to protect a patient's electronic PHI. The security rules generally require reasonable and appropriate safeguards and procedures to verify and protect patient health information that is held or transferred in electronic form. Yet data breaches continue, imposing significant costs on providers and loss of privacy for affected patients. For example, between 2009 and 2017, two thousand one hundred eighty-one (2,181) healthcare data breaches were reported (*https:*//*www.hipaajournal.com*/*healthcare-data-breach-statistics*/)*.* Those breaches resulted in the theft and exposure of a staggering one hundred and seventy-six million, seven hundred and nine thousand, three hundred and five (176,709,305) healthcare records, which equals more than 50% of the U.S. population (*id*).

Portable smart cards are sometimes used to transport and protect a patient's sensitive data from unauthorized access, tampering, or theft. Conventional methods for authenticating such smart cards require the use of passwords or keys which are common to all the smart cards. Known smart card readers send a global password stored in the system to the smart card. The known smart cards then verify the global password with an internally stored global password. However, the known systems and methods require the medical system to maintain a copy of the common global password. If one of the smart card's password is compromised, then the smart cards and related data used in the known systems and methods may become compromised. Hackers can also more easily tamper vulnerable known systems and methods using a compromised password obtained by an unauthorized third party smart card reader, such as a skimmer. Such breaches can result in patient and configuration data becoming susceptible to class attack and large data loss.

Hence, systems and methods can be provided that improve upon known protecting schemes that guard against unauthorized and unintentional access to medical systems. Encrypting data such as personal health information (PHI) before writing to a smart card and decrypting after reading to ensure that data remains confidential can be provided. An authentication mechanism between a smart card and a reader to reduce the possibility of tampering using third party smart card readers can also be provided. An automated smart card password generation and authentication system to minimize risk of breach in a distributed healthcare environment, ecosystem, or provider network can be further provided. A unique password to each smart card in a trackable manner can be provided. Avoiding the storage of the unique passwords in a system can be provided. Denying access to a third-party smart card reader attempting to access data stored in a smart card can also be provided. Avoiding a class attack, so that a compromise of one smart card's password will not affect other smart cards in the system can be provided. Systems and methods suitable for use in various medical systems including, but not limited to, dialysis can also be provided.

### SUMMARY OF THE INVENTION

The first aspect of the invention relates to a secure medical system for transmitting personal health information. In any embodiment, the secure medical system can include a smart card; a smart card reader, and a microprocessor; wherein the smart card has a secure computer readable memory having memory allocated to store a first password verifiable by the smart card reader and a second password generated by the smart card reader, and a file directory containing personal health information and a unique ID, wherein the smart card reader can use an algorithm based at least partly on the unique ID to generate the second password; the microprocessor having instructions for verifying a stored second password against a received second password sent by the smart card reader, the microprocessor providing access to the file directory containing data, if the stored second password matches the received second password.

In any embodiment, the smart card can further include an antenna, a capacitor, and a non-volatile memory unit.

In any embodiment, the antenna can communicate with the smart card reader by transmitting and/or receiving radio frequency or wireless signals based on instructions from the microprocessor.

In any embodiment, the smart card can further include a subscriber identification module chip, also known as a SIM chip, which can make direct electrical contact with the smart card reader to transmit data.

In any embodiment, the first password can be a factory-preset password.

In any embodiment, the unique ID can be any one of a serial number, a globally unique identifier (GUID), or a hashed number.

In any embodiment, the personal health information can include any one or more of a name, age, gender, height, weight, patient ID, prescription data, treatment data, device configuration, or any other medically relevant patient data not limited to patient history, prior therapy session data, genetic data, and the like.

In any embodiment, the first password can be overwritten by the second password.

The features disclosed as being part of the first aspect of the invention can be in the first aspect of the invention, either alone or in combination, or follow any arrangement or permutation of any one or more of the described elements. The described combinations can solve one or more one-limiting problems related to smart cards requiring the use of passwords or keys which are common to all the smart cards and storage of passwords by a smart card reader.

The second aspect of the invention is drawn to a smart card reader for use in a medical system. In any embodiment, the smart card reader can include a microprocessor having instructions for transmitting and receiving radio frequency or wireless signals and an algorithm for generating a second password; an antenna for communicating with a smart card, where the antenna can transmit and/or receive radio frequency or wireless signals based on instructions from the microprocessor; a secure computer readable memory storing a first password verifiable by the smart card and memory allocated for a unique ID received from the smart card; and the microprocessor further including instructions for generating the second password based at least partly on the unique ID received from the smart card, instructions for either transmitting and storing the second password on the smart card for initial preparation of the smart card, instructions for transmitting the second password to access a secured file directory containing data on the smart card, and instructions for discarding the second password.

In any embodiment, the algorithm can be at least partly based on partial secret and number of iterations of a pseudo-random function.

In any embodiment, the number of iterations of the pseudo random function can be greater than 1.

In any embodiment, the partial secret can be identical across medical devices.

In any embodiment, the pseudo-random function can be HMAC-SHA1, HMAC-SHA2, HMAC-SHA3, or PBKDF2.

In any embodiment, the length of the second password can be at least 4 bytes.

In any embodiment, the smart card reader can have a slot suitable for receiving the smart card, which can allow the smart card reader to transmit data to and/or from the smart card by making direct electrical contact.

In any embodiment, the smart card reader can be in electrical communication with any one or more of a desktop computer, a laptop computer, or any other medical system.

The features disclosed as being part of the second aspect of the invention can be in the second aspect of the invention, either alone or in combination, or follow any arrangement or permutation of any one or more of the described elements. The described combinations can solve one or more one-limiting problems related to storing or maintaining a copy of a common global password.

The third aspect of the invention is drawn to an automated smart card authentication system for use in a medical system. In any embodiment, the system can include a smart card having memory allocated for storing a first password, a second password generated by an algorithm based on a unique ID, the unique ID, and personal health information; a smart card reader programmed to receive the first password stored in the smart card, wherein the smart card reader can generate a second password using the algorithm based at least partly on the unique ID of the smart card, store the second password on the smart card and discard the second password from the smart card reader; where the smart card reader is programmed to generate the second password on each read of the smart card using the algorithm; where the smart card can grant access to data if the second password generated by the smart card reader matches the second password stored in the smart card, and the smart card reader can discard the second password.

In any embodiment, the smart card reader can transmit the data from the smart card to other medical devices connected to the smart card reader.

In any embodiment, the second password generated by the smart card reader can be stored on the smart card. In any embodiment, the second password is not stored anywhere else on the system.

The features disclosed as being part of the third aspect of the invention can be in the third aspect of the invention, either alone or in combination, or follow any arrangement or permutation of any one or more of the described elements. The described combinations can solve one or more one-limiting problems related to smart card readers storing or maintaining a copy of a common global password.

The fourth aspect of the invention relates to a method for automated smart card authentication for use in a medical system. In any embodiment, the method can include the step of accessing a smart card using a first password and a unique ID stored on the smart card, wherein the unique ID is used to generate a second password by a smart card reader, wherein the smart card reader uses an algorithm based at least partly on the unique ID to generate the second password wherein the second password is stored on the smart card and is discarded and not stored by the smart card reader.

In any embodiment, the method can include the step of re-generating the same second password by the smart card reader using the unique ID of the smart card and upon verification of the same second password on the smart card, access a file directory containing personal health information on the smart card.

The features disclosed as being part of the fourth aspect of the invention can be in the fourth aspect of the invention, either alone or in combination, or follow any arrangement or permutation of any one or more of the described elements. The described combinations can solve one or more one-limiting problems related to methods requiring the storage or maintaining a copy of a common global password.

The fifth aspect of the invention relates to a smart card. In any embodiment, the smart card can have a secure computer readable memory having memory allocated to store a first password verifiable by a smart card reader and a second password generated by the smart card reader; a file directory containing personal health information, and a unique ID, wherein the smart card reader uses an algorithm based at least partly on the unique ID to generate the second password; and a microprocessor having instructions for verifying a stored second password against a received second password sent by the smart card reader, the microprocessor providing access to the file directory containing data, if the stored second password matches the received second password.

In any embodiment, the smart card can have an antenna, a capacitor, and a non-volatile memory unit.

In any embodiment, the smart card can have a subscriber identification module chip, wherein the subscriber identification module chip makes direct electrical contact with the smart card reader to transmit data.

In any embodiment, the first password can be overwritten by the second password.

In any embodiment, the personal health information can include any one or more of a name, age, gender, height, weight, patient ID, prescription data, treatment data, and device configuration.

The features disclosed as being part of the fifth aspect of the invention can be in the fifth aspect of the invention, either alone or in combination, or follow any arrangement or permutation of any one or more of the described elements. The described combinations can solve one or more one-limiting problems related to smart card readers storing or maintaining a copy of a common global password.

### BRIEF DESCRIPTION OF THE DRAWINGS

FIG. 1 is a flow chart of a smart card usage.
FIG. 2 is a flow chart of an algorithm used to generate a unique password.
FIG. 3 is an example of a smart card file directory.
FIG. 4A is a flow chart showing an initial setup procedure for a smart card.
FIG. 4B is a flow chart for accessing the smart card after the initial setup.
FIG. 5 is an exemplary embodiment of a wireless smart card.
FIG. 6 is an exemplary embodiment of a cabled smart card.
FIG.'s 7A-C are exemplary embodiments of a smart card reader.
FIG. 8 is an exemplary embodiment of a smart card reader as a part of a computer.
FIG. 9 is an example of the smart card used in clinical setting with multiple therapy devices and multiple patients.

### DETAILED DESCRIPTION

Unless defined otherwise, all technical and scientific terms used have the same meaning as commonly understood by one of ordinary skill in the art.

The articles "a" and "an" are used to refer to one to over one (i.e., to at least one) of the grammatical object of the article. For example, "an element" means one element or over one element.

The term "access" or the step of "providing access" or "granting access" can refer to allowing authorization to create, read, list, display, or update information or data stored in a memory, processor, device, or component.

The term "algorithm" can refer to the broadest interpretation of a process, mathematical operation, or method of any type that can be used to generate data, files, parameters, passwords, text, or information.

The term "allocated" can refer to any process that assigns a block of memory for data or for a program.

The term "antenna" can be any component capable of sending or receiving electromagnetic waves or signals.

The term "authentication system" can refer to any system that allows for identification of a particular component by any suitable means known to those of skill in the art.

The term "automated" refers to the broadest interpretation of any algorithm or process that can be performed, in part or wholly, by a machine or apparatus. The operations involved in the "automated" algorithm or process can sometimes occur without human intervention or with partial support by a human. No restriction is placed on the extent to which the algorithm or process is automated wherein any portion of the algorithm or process can be automated.

The phrase "based at least partly on" can refer to a key element or feature, but also optionally include any other non-key elements or features.

A "byte" can refer to a unit of digital information that commonly consists of eight bits.

A "capacitor" can refer to a component capable of storing electric energy.

The term "comprising" includes, but is not limited to, whatever follows the word "comprising." Use of the term indicates the listed elements are required or mandatory but that other elements are optional and may be present.

A "computer readable memory" can refer to any memory which can be accessed by a computer to transmit information stored with the memory.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The term "contain" or "containing" refers to any component from which information can be obtained. The information can include but is not limited to patient information, system information and component information.

The terms "control," "controlling," or "controls" refers to the ability of one or more components to direct the actions of a second set of one or more components.

The term "data" can refer to any quantity, text, character, or symbol containing or representing information of any type. In general, a computer can perform operations on the data, and the data recorded and stored on any one of magnetic, optical, electrical, memory, or mechanical recording media, and transmitted in the form of digital electrical signals.

The tern "desktop computer" can refer to any machine containing a processor. The desktop computer is capable of being electrically connected to other components or devices.

The term "device configuration" can refer to a set of data or information capable of modifying or instructing how a device should perform in a particular condition or environment.

"Dialysis" is a type of filtration, or a process of selective diffusion through a membrane. Dialysis removes solutes of a specific range of molecular weights via diffusion through a membrane from a fluid to be dialyzed into a dialysate. During dialysis, a fluid to be dialyzed is passed over a filter membrane, while dialysate is passed over the other side of that membrane. Dissolved solutes are transported across the filter membrane by diffusion between the fluids. The dialysate is used to remove solutes from the fluid to be dialyzed. The dialysate can also provide enrichment to the other fluid.

The terms "discard" or "discarding" can refer to deleting, either permanently or temporarily, any data or information from any memory, computer, or system.

The phrase "direct electrical contact" can refer to a part of a device in contact or attaching to a part of another device. Such contact can optionally provide for the contacted parts to transmit data or information via electrical signal, or any other means.

The terms "electrical communication," "electrically communicate," "electrically communicating," and the like can refer to the ability to transmit electronic data, instructions, information wirelessly, via direct electrical connection, or any other electrical transmission means between one or more components.

The term "factory-preset" can refer to any information or data stored on a component or device at the time of manufacture.

The "file directory" can refer to any architecture, format, digital pathway, or storage structure where information or data can be stored in electronic form.

The terms "generate," "generating," or "to generate" can refer to any one of creating, determining, or calculating any parameter, file, or feature (e.g., password) based on any input. Similarly, the terms "re-generate," "re-generating," or "to re-generate" can refer to any one of recreating, re-determining, or re-calculating any parameter, file, or feature (e.g., password) based on any input. For example, a microprocessor can generate or re-generate a unique ID using an algorithm stored in a microprocessor.

A "globally unique identifier" or "GUID" can refer to a 128-bit number used to identify information of any suitable type.

The phrase "value greater than one" means any integer greater than one.

A "hashed number" can refer to a number transformed into another form by a particular function.

A "hash-based message authentication code," "keyed-hash message authentication code," or "HMAC" can be used to confirm that a message's content or its sender information has not been modified by using cryptographic hash function and a secret cryptographic key.

The terms HMAC-SHA1, HMAC-SHA2, HMAC-SHA3, PBKDF2, et al. refer to file transfer protocols using a particular type of hash function such as SHA1, SH2, SH3, et al.

The term "identical" can refer to any component, data, process, algorithm, information that is the same in every detail or aspect.

An "initial preparation" can refer to a set of process setting up any component, to a state suitable for a subsequent use. In one non-limiting example, a smart card, which initially comes with a first password, may be given a second password to be stored, which allows the smart card to be subsequently used in an automated manner.

The terms "instruction" or "instructions" can refer to one or more steps directing any one or combination of a circuit, software, or a processor to perform one or more function or process.

The term "laptop computer" can refer to a portable computing platform with one or more microprocessor and memory, both of any type.

The term "match" can refer to any condition or state wherein a feature or file is identical to another feature or file. In general, the term can be used in the context of passwords wherein one entered password is identical to another stored password for verification.

The term "medical system" can be a set of one or more interoperable components capable of any one or more of sensing one or more medical parameters, performing or delivering a medical therapy of any type, and/or storing medical information.

The term "memory" is a device for recording digital information that can be accessed by any microprocessor, such as RAM, Dynamic RAM, microprocessor cache, non-volatile memory such as EEPROM, Flash memory or any other similar memory device.

A "non-volatile memory unit" can refer to any type of memory which allows data to be stored or held even if power to the memory unit is turned off.

The phrase "number of iterations" can refer to a count of a pseudo-random function being executed.

The term "overwritten" or to "overwrite" refers to replacing information saved in a computer readable medium with different information.

The term "partial secret" can refer to any part of a data or number which can be stored in a device or in memory such that data is not readily visible or known to a device user. The data or number can be identical across any type of system or devices.

The term "password" refers to information required by a component or system for gaining access to the component or system.

The term "password-based key derivation function version 2.0" or "PBKDF2" can refer to a type of key derivation function that utilizes a pseudo random function to derive a key that can be used in subsequent functions.

A "patient" or "subject" can be a member of any animal species, preferably a mammalian species, optionally a human. The subject can be an apparently healthy individual, an individual suffering from a disease, or an individual being treated for a disease. In certain embodiments, the patient can be a human, sheep, goat, dog, cat, mouse or any other animal.

The terms "patient identifier" or "patient ID" can refer to information capable of distinguishing one patient from another.

The terms "prescription" or "patient prescription" can refer to one or more system settings for performing a therapy. In one non-limiting embodiment, the prescription can be directed to any parameter used in setting a dialysis session for a patient.

The term "personal health information" can refer to any health data concerning an individual. The term can be used in the context of a particular patient where the information can include, but is not limited to, patient name, patient ID, prescription data, treatment data, genetic and phenotypic data, specific population data, and specific events having occurred during a prior medical treatment or occurring during an on-going treatment.

The term "pseudo-random function" can refer to a function emulating a completely random function. In one non-limiting embodiment, an efficient algorithm cannot distinguish between a completely random function and a pseudo-random function.

The term "processor" or "microprocessor" as used is a broad term and is to be given an ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. In any embodiment of the first, second, third, and fourth invention, the terms can include ROM ("read-only memory") and/or RAM ("random-access memory") associated therewith.

The term "programmed," when referring to a processor, can mean a series of instructions that cause a processor to perform certain steps.

"Radio frequency" refers to electromagnetic radiation with a frequency from about 3 kHz to about 300 GHz.

The terms "read," "to read," "reading," and the like, in referencing a computer process, means the act, state, or process of receiving information or data by any known electrical or digital means.

The term "readable" refers to a memory component that contains information obtainable by another component.

The terms "received," "to receive," "receiving," and the like, in reference to an object, can refer to allowing ingress or generally allowing any object, such as a smart card, to be positioned or seated into a space where the object is placed. The terms if referring to an electrical transmission or communication means obtaining an electrical signal of any type.

The phrase "receive data" generally refers to the process of obtaining information or data from any source by any means including wireless, direct contact, electrical contact, energy transfer, magnetic resonance, or any other known means.

The term "secure" as applied to electronic data and components, generally refers to a state or condition in which the data or component has received any type of protective digital measures to prevent unauthorized access.

A "Secure Hash Algorithm 1, 2, or 3" or "SHA-1, 2, or 3" can refer to a type of cryptographic hash function.

A "serial number" can be a unique identifying number assigned to a component.

A "slot" is any opening on any device or machine for reversibly receiving another component.

A "smart card" can be a substantially rectangular and planar portable component that can contain either a memory and a microprocessor, or both. Other non-limiting components such as antennas and receivers can also be contained on the smart card.

A "smart card reader" is a component that is capable of transmitting information to and from a smart card. The transmission can occur by any means including wireless, direct contact, electrical contact, energy transfer, magnetic resonance, or any other known means.

The terms "stored," "to store," or "storing" refer to a process for assigning data to a memory. Optionally, the data can be retrieved later.

The terms "subscriber identification module chip" or "SIM chip" can refer to an integrated circuit. The SIM chip can in certain embodiments securely store information required to identify or authenticate a device or component.

The terms "transmission," "to transmit," "transmitting," and the like can refer to the ability to send electronic data, instructions, information wirelessly, via direct electrical connection, or any other electrical communication means between one or more components.

"Treatment data" can refer to any information regarding a patient's prior medical treatment, including, but not limited to, prescriptions or dosing, history of prior treatment, status of medical components and settings for a medical system for the prior treatment, events occurred during the prior treatment, medical outcome of the treatment, and the like.

A "unique identifier" or "unique ID" can be information or data capable of distinguishing one feature or component from other features or components.

"Verify", "verifying" or "verifiable" refers generally to a process for comparing two or more digital features or files. In one non-limiting embodiment, passwords to determine whether the two features or files can be matched or will match each other can be verified. The verification process can generally include any one or more of algorithms, electronic methods, and defined procedures to determine the matching or matchability of the two or more digital features of files.

A "wireless signal" can refer to electromagnetic or radio wave which can transfer information between two devices that are not directly connected to each other by wired contact.

### Smart Card Access Control System

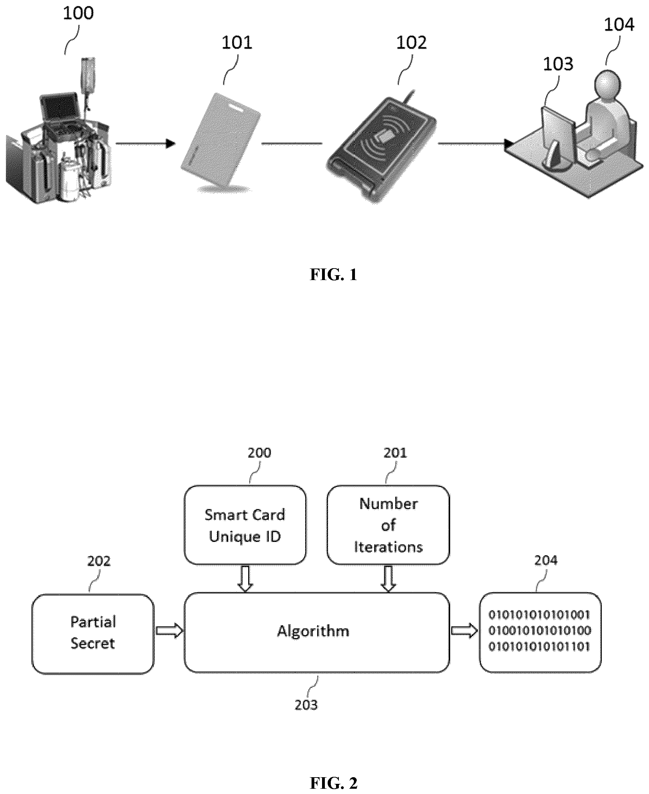

FIG. 1 illustrates a non-limiting embodiment of a smart card **101** used in a medical system 100. The system can generate a unique password associated with each smart card and compute the unique password each time when access is required, resulting in an automated smart card password generation and authentication system. Smart card readers that are not part of the system cannot access the smart card file directory because smart card readers that are not part of the system cannot compute the unique password. The systems and methods can also avoid class attack where a single compromised does not affect other smart cards. The automated smart card password generation and authentication system can be implemented by software applications that can perform required routines for initiating, executing, and completing the password generation and authentication steps.

The medical system **100** can include, but is not limited to cardiac monitoring, hemodialysis, peritoneal dialysis, or patient monitoring. In one non-limiting embodiment, the medical system **100** is directed to a portable hemodialysis device. The smart card **101** can store personal health information (PHI) including sensitive patient data such as prescription, treatment data, or device configuration data. The smart card **101** can also store data such as a patient's biometric or genetic information. The patient data can originate from the medical system **100** and be stored in smart card **101.** A smart card reader **102** can access the stored data in smart card **101** upon making contact either by direct electrical contact, cabled contact, wireless transmission, magnetic resonance, impedance, or any other kind of known transmission means. Non-limiting examples of wireless transmission can include any desired wired or wireless technology, including, for example, cellular, WAN, wireless fidelity (Wi-Fi), Wi-Max, WLAN, and the like. Still other non-limiting technologies can include BLUETOOTH, BLUETOOTH low energy (BLE), near field communication (NFC), Zigbee, RF4CE, WirelessHART, 6LoWPAN, Z-Wave, ANT, and the like. The transmitter/receiver architecture of the smart card reader **102** and smart card **101** can vary depending on the type of transmission protocol. For example, the smart card reader **102** and smart card **101** can be configured to perform one or more different types of transmission protocols (e.g., BLUETOOTH, BLE, NFC, Wi-Fi, Zigbee, etc.). In other embodiments, the smart card reader **102** and smart card **101** can include a plurality of different transmitters/receivers that are respectively configured to perform different types of wireless communication protocols.

In one non-limiting embodiment, the smart card **101** and the smart card reader **102** can each contain an antenna so that smart card **101** and smart card reader **102** can wirelessly communicate with each other by transmitting and receiving data. Alternatively, the smart card reader **102** can have a slot such that smart card **101** can be inserted into smart card reader **102** to communicate by direct electrical contact with smart card **101** to transmit and receive data. Alternatively, the smart card reader **102** can communicate with the smart card **101** by having a groove where the smart card **101** can be swiped, if the smart card **101** has a magnetic strip. The use of the smart card **101** can be automated so that a user is not required to manually type a password to gain access.

The smart card reader **102** can be connected to a computer **103.** In certain embodiments, the computer **103** can be a desktop or a laptop computer. The computer **103** can display the data stored in the smart card **101** to a medical professional **104** if the smart card reader **102** has gained access to the smart card **101.** Using the computer **103,** a medical professional **104** can update or modify a dosage, prescription, therapy parameters, patient data, or any other desired data. The smart card reader **102** can also be connected to a laptop computer, where the laptop computer performs a similar function as described for the desktop computer **103.** Alternatively, the smart card reader **102** can be connected to another medical system **100.** If the smart card reader **102** is connected to a medical system **100** and the smart card reader **102** has gained access to the smart card **101** that stores a device configuration, the medical system **100** can download and access and update the device configuration. If the smart card reader **102** is connected to the medical system **100** and the smart card reader **102** can gain access to the smart card **101,** and the medical system **100** can also modify performance based on the patient data stored in the smart card **101.** Alternatively, the medical system **100** can automatically update or edit the data stored in the smart card **101** while connected to the smart card reader **102.** Alternatively, a patient can also use computer **103** to access his or her own patient data. In certain embodiments, the smart card reader **102** can be a standalone device and does not need to be connected to a computer **103** or a medical system **100** to be operational. Alternatively, a computer **103** or a medical system **100** can be constructed so that a smart card reader **102** is integrated as a part of the devices, as opposed to being attachable and detachable to the computer **103** or the medical system **100.**

The smart card reader can use an algorithm to generate a password. FIG. 2 illustrates a non-limiting flow chart for an algorithm **203** used to generate a password **204.** To generate a unique password **204** for each smart card, a smart card unique ID **200** can be entered into algorithm **203.** A partial secret **202,** which is stored in medical devices and shared among them, can be incorporated into algorithm **203** to generate a unique password **204.** The partial secret **202** can be varied depending on a type of a medical device or location. The partial secret **202** may not be known or visible to smart card or medical device users. In certain embodiments, a pseudo-random function can be incorporated into algorithm **203.** Pseudo-random functions can include HMAC-SHA1, HMAC-SHA2, HMAC-SHA3, and other pseudo-random functions known to those of skill in the art. One of skill in the art will appreciate that additional types of pseudo-random functions can be incorporated into algorithm **203.** Moreover, the systems and methods can have different combinations of cryptographic hash functions or one type of cryptographic hash function. The number of iterations of the pseudo-random function **201** can also be inputted into algorithm **203.** In certain embodiments, the number of iterations of the pseudo-random function **201** can be any value greater than one. The practical maximum number of iterations of the pseudo-random function can be determined by the processing time and processing capability of the hardware and processors. The number of iterations of the pseudo-random function **201** can refer to an execution count of the pseudo-random function. If the number of iterations of the pseudo-random function **201** is constant, the pseudo-random function can yield the same outcome. The number of iterations of the pseudo-random function **201** can be varied depending on a type of a medical device or location. Any one or more of the smart card unique ID **200,** the number of iterations of pseudo-random function **201,** and the partial secret **202** can be incorporated into algorithm **203** to generate a unique password **204.** The algorithm **203** may be a password-based key derivation function version 2.0 (PBKDF2), or another algorithm designed for generating a password **204.** The PBKDF can optionally be based on IETF RFC 2898. A person skilled in the art will understand and appreciate other known algorithms that can be utilized to create password **204.** The password **204** length can be at least 4 bytes.

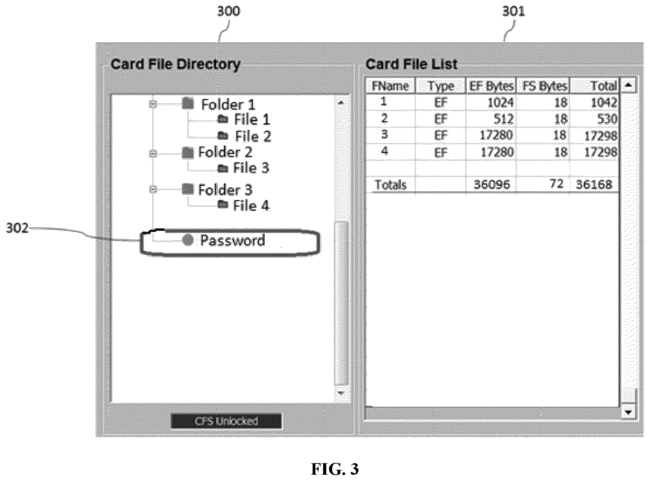

If a smart card reader is connected to a computer and gains access to a smart card, the computer can display data stored within the smart card. FIG. 3 illustrates a non-limiting example of the data that can be accessed or displayed on a computer screen. A card file directory **300** can list folders and files stored in a smart card. In certain embodiments, a card file list **301** can appear next to the card file directory **300** and show detailed information of the stored data. The detailed information can include personal health information. A user who has gained access to the data can update or modify the information. The user can also download the data from the smart card to another device. The card file directory **300** can also list a first password **302.** The first password **302** can be used to allow the system to store a second, unique password. The second password can be used to gain access to the smart card. The first password **302** can be a factory-preset password generated or installed at or around the time of initial manufacture as known to those of skill in the art. The first password **302** can be a password that verifies a password sent by a smart card reader. The first password **302** can be different from the second password, which is generated from the algorithm in FIG. 2. The first password **302** can be protected from unauthorized attempts to access, erase or modify the first password **302.**

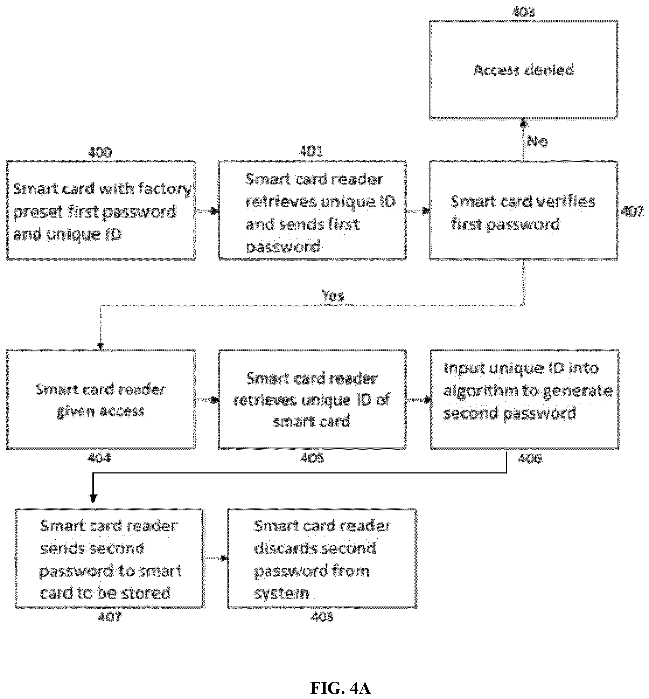

FIG. 4A illustrates a non-limiting flow chart for an initial preparation of a smart card for operational use in a medical system. The smart card can be initially prepared with a factory-preset first password and a unique ID, as shown in a step **400.** In step **401,** a smart card reader can retrieve the unique ID and send a first password to the smart card. In certain embodiments, the unique ID can be the information on the smart card that can be read or accessed without authorization. In step **402,** the smart card can verify the first password with the first password stored in the smart card. In certain embodiments, the first password can be required for the smart card reader to store a second password on the smart card. If the two first passwords do not match, access can be denied as shown in step **403,** or the smart card can prevent the smart card reader from setting a second password. On the other hand, if the two first passwords match, the smart card can give access to the smart card reader in step **404,** allowing the smart card reader to store a second password on the smart card. In step **405,** the smart card reader can retrieve the smart card's unique ID. In certain embodiments, access to the unique ID can be independent of the verification process described in step **402.** Alternatively, access to the unique ID can occur after the smart card reader is given access. After the smart card reader retrieves the unique ID, the smart card reader can input at least the unique ID into an algorithm to generate a second password, as shown in step **406.** In step **407,** the smart card reader can send the generated second password to the smart card to be stored. In certain embodiments, the smart card reader can overwrite the first password with the second password. Alternatively, the smart card can include memory allocated for storing both the first password and the second password. After the second password is stored in the smart card, the smart card reader can discard the second password from the smart card reader, a computer which may be connected to the smart card reader, or any other authentication system, as shown in step **408.** After the steps described in FIG. 4A, the smart card can erase the factory-preset first password to ensure that the smart card cannot be re-initialized by or tampered with by an unauthorized program or user. After the second password is stored in the smart card, the unique ID of the smart card remains freely accessible unlike other stored data, which is protected from unauthorized attempts to access.

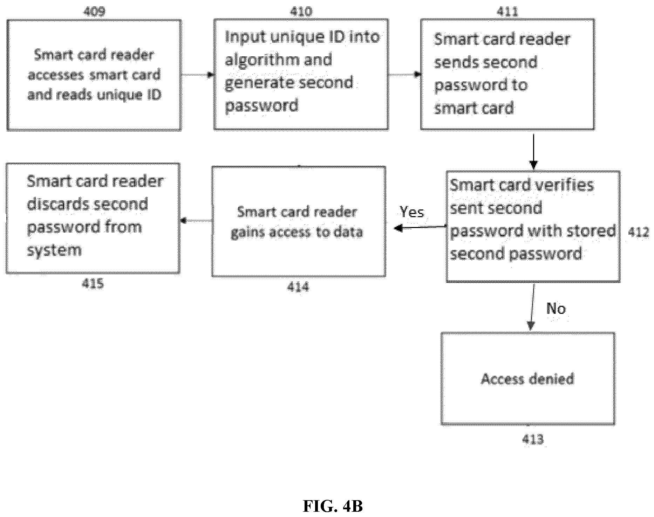

FIG. 4B illustrates a non-limiting flow chart for controlling access to the smart card after an initial preparation process. The smart card reader can access a smart card reader and read a unique ID of the smart card in step **409.** At this point, the smart card reader does not yet have access to any other data stored in the smart card but the unique ID. In step **410,** the smart card reader can input at least the unique ID into an algorithm and generate a second password using the same algorithm as in FIG. 4A. Then, in step **411,** the smart card reader can send the generated second password to the smart card. In step **412,** the smart card can verify the sent second password with a stored second password. If the second passwords do not match each other, the smart card can deny access to the smart card reader, as shown in step **413.** If the second passwords match each other, then the smart card reader can be permitted to access data stored in the smart card, as shown in step **414.** The smart card reader can access the data stored in the smart card, and update or edit the data. The smart card reader can also download the data to any device connected to the smart card reader. In step **415,** after the smart card reader sends the second password to the smart card for verification, the smart card reader can discard the generated second password from the smart card reader, a computer connected to the smart card reader, or any other authentication system except the smart card. In certain embodiments, the generated second password can be discarded, regardless of whether the smart card reader has gained access to the smart card. The smart card can also require the smart card reader to send both the first password and the second password for verification.

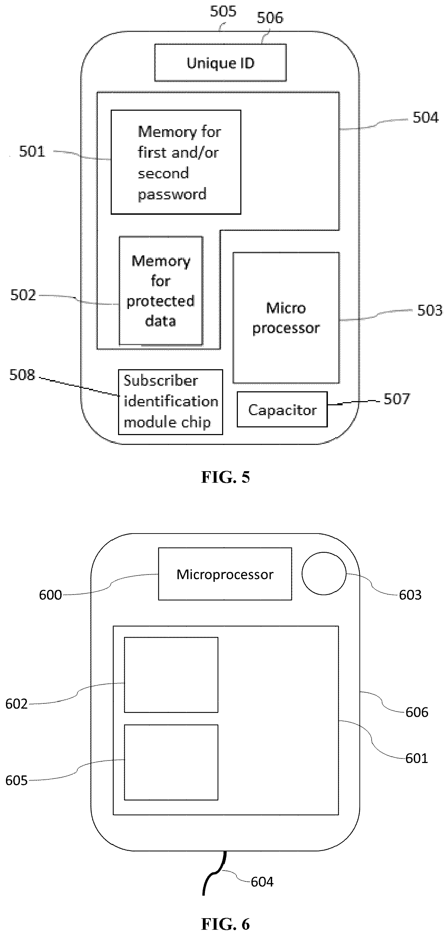

FIG. 5 illustrates a non-limiting embodiment of a smart card **505.** A smart card **505** contains the unique ID **506** of FIG. 2 to provide each smart card an individual and unique identification. The unique ID **506** can be read by a smart card reader and inputted into an algorithm to generate a second password. Non-limiting examples of the unique ID **506** can be a serial number, a globally unique identifier, a hashed number, or any other type of identification source known to those of ordinary skill. The smart card **505** can contain a computer readable memory **504,** where various types of data can be stored. In certain embodiments, the computer readable memory **504** can be a non-volatile memory unit. The unique ID **506** can be stored separately from the computer readable memory **504.** Alternatively, the unique ID **506** can be stored in the computer readable memory **504.** The computer readable memory **504** can include memory space **501** allocated to store a first password and/or second password. The first password can initially be stored on the smart card **505** when the card is first manufactured or produced in a factory. The memory space **501** initially containing the first password can be protected from any attempt to erase or modify the first password. The first password can be erased from the computer readable memory **504** after a second password is saved into the memory space **501.** In certain embodiments, a memory space **501** allocated for the second password can be initially empty. When the smart card reader reads unique ID 506 and generates the second password, the smart card reader can send the second password to the smart card 505, which stores the second password to the memory space 501 allocated for the second password. In other embodiments, only a single memory space 501 is used. If the smart card reader generates the second password, the smart card reader can overwrite the first password with the second password in the memory space 501. In certain embodiments, once the second password is stored in the memory space 501 allocated for the second password, the second password can be protected from any attempt to erase or modify the stored second password.

After the computer readable memory **504** has stored the second password on the memory space **501** allocated for the second password, the smart card **505** can grant access to the smart card reader for data **502** when the smart card **505** receives and verifies the second password sent by the smart card reader. The memory space for protected data **502** can contain personal health information such as prescription or treatment data, as well as biological data of a patient and patient ID. In certain embodiments, protected data can include configuration for a medical device or system as well as any data a medical professional may find useful in treating a patient. The smart card **505** contains a microprocessor **503,** which can execute a set of programmed directions. The microprocessor **503** can be programmed to verify a first password sent by the smart card reader against the stored first password. The microprocessor **503** can be programmed to store the second password, generated and sent by the smart card reader, to the memory space **501** allocated for the second password, after the smart card reader access is verified with the first password. The microprocessor **503** can be programmed to grant access to the protected data **502** to the smart card reader after verification of the second password, allowing the smart card reader to read or update the protected data **502.** The microprocessor **503** can be programmed to execute other functions of the smart card **505** necessary for the card to work properly. A person skilled in the art will understand and appreciate other suitable functions of the smart card **505** that can be programmed into the microprocessor **503** and executed for specific applications.

In certain embodiments, the smart card **505** can contain an antenna, a capacitor **507,** a non-volatile memory unit, and other parts necessary for the smart card **505** to function properly. An antenna can transmit and receive radio frequency or wireless signals based on instructions from the microprocessor **503.** The smart card **505** can optionally contain a subscriber identification module chip **508.** The subscriber identification module chip **508** can substitute the function of an antenna and transmit and receive data if the subscriber identification module chip **508** is in direct electrical contact with a smart card reader designed to interact with the subscriber identification module chip **508.** The smart card **505** can have other elements to transmit and receive data to and from a smart card reader. One example can be a magnetic strip.

FIG. 6 illustrates a non-limiting exemplary embodiment of a smart card reader **606.** The smart card reader **606** can include a microprocessor **600,** a memory **601,** and an antenna **603.** The smart card reader **606** can be connected to a computer or a medical system by an electric cable **604** or wirelessly. Alternatively, the smart card reader **606** can be a part of a computer or a medical system. Alternatively, the smart card reader **606** can operate without being connected to a computer or a medical system. The microprocessor **600** can give instructions to the antenna **603** to wirelessly communicate with a smart card and send a first password **602** to the smart card for verification. The first password **602** is stored in the memory **601.** The microprocessor **600** can contain an algorithm, which can incorporate at least a unique ID of the smart card, partial secret **605,** and a number of iterations of a pseudo-random function to compute a second password. The partial secret **605** can be varied depending on a type of a medical device or location. The partial secret **605** may not be known or visible to smart card or medical device users. If the smart card reader **606** gains access to the smart card, personal health information stored in the smart card can be downloaded and stored in the memory **601.** Alternatively, the smart card reader **606** can relay the protected personal health information from the smart card to the computer or the medical device to which the smart card reader **606** is connected.

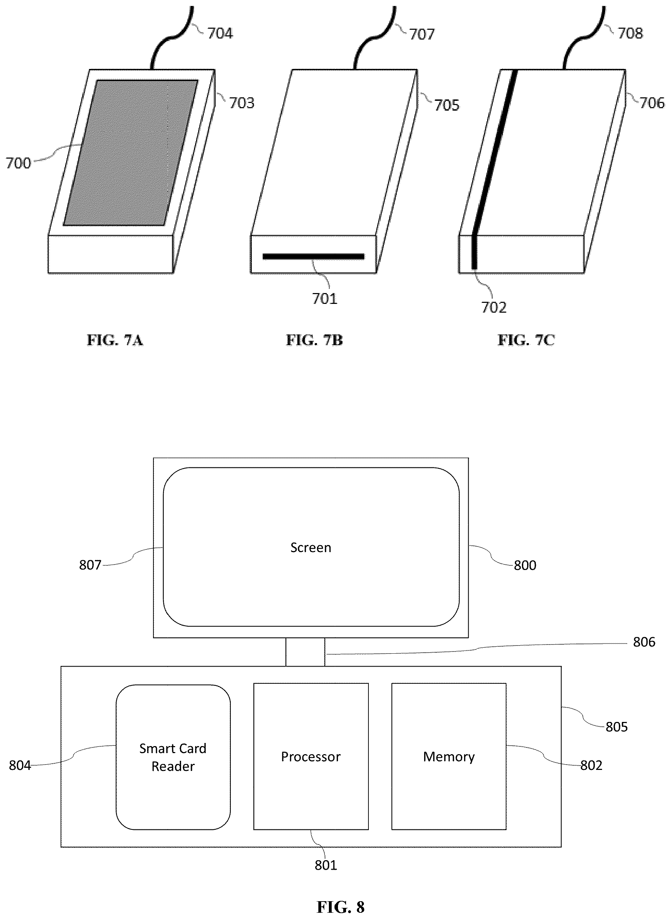

FIG.'s 7A, 7B, and 7C illustrate three non-limiting examples of a smart card reader **703.** In FIG. 7A, the smart card reader **703** can have an electric cable **704** which connects the smart card reader **703** to a computer or a medical device, and a contact area **700,** which can contain an antenna underneath the contact area **700,** enabling the smart card reader **703** to communicate wirelessly with a smart card when the smart card is placed on or close to the contact area **700.** Alternatively, the smart card reader **703** can be integral to a medical device, computer, or any other component. In any embodiment, the smart card readers can use Radio Frequency Identification (RFID) technology or similar technology known to those of skill in the art. RFID technologies can use radio waves to automatically identify components. The RFID technology systems can be used to manage electronic access and control of the described systems and methods. RFID technology can use microchips that are in communication with a transponder, commonly referred to as a marker of RFID or RFID tag. An antenna, sometimes packaged with a transceiver and decoder, can be used to transmit data the transceiver. The transceiver and/or decoder can receive and convert the radio waves reflected from the RFID marker into digital information that can then be switch to any processor or computers for processing. Using any suitable known wireless or contactless technology, the smart card can be placed on or adjacent to the contact area **700** so that the smart card reader **703** can communicate with the smart card. The smart card is not required to make any direct electrical contact with the contact area **700** to communicate with the smart card reader. The antenna inside the smart card reader **703** can communicate by transmitting and receiving radio frequency or wireless signal to and from the smart card.

In FIG. 7B, the smart card reader **705** can have a slot **701** where a smart card (not shown) can be inserted to allow the smart card reader **705** to communicate with the smart card. Optionally, the smart card reader **705** can have an electric cable **707** which can connect the smart card reader **705** to a computer or a medical device. Alternatively, the smart card reader **705** can be integral to a medical device, computer, or any other component. The smart card can make direct electrical contact with the smart card reader **705** by being inserted into slot **701.** The smart card can be left inserted in slot **701** for the smart card and the smart card reader **705** to communicate. The smart card can also be taken out from the slot **701** after the smart card reader **705** reads data from the smart card. The smart card can contain a subscriber identification module chip to provide the direct electrical contact between the smart card and the smart card reader **705** when the smart card is inserted into the slot **701.** Alternatively, other types of chips or circuits known to a person skilled in the art can be used to provide the direct electrical contact between the smart card and the smart card reader **705.**

In FIG. 7C, the smart card reader **706** can have a groove **702** where a smart card can be swiped to allow the smart card reader **706** to communicate with the smart card. Optionally, the smart card reader **706** can have an electric cable **708** which can connect the smart card reader **706** to a computer or a medical device. Alternatively, the smart card reader **706** can be integral to a medical device, computer, or any other component. The smart card can contain a magnetic strip so that the smart card reader **706** can communicate with the smart card when the smart card is swiped into the groove **702.** Alternatively, other means known to a person skilled in the art can be used instead of the magnetic strip so that the communication between the smart card and the smart card reader **706** employing the groove **702** is provided.

The smart card readers **703** and **705-706** are not limited to any particular type of communication components as shown in FIG.'s 7A - 7C. In a certain embodiment, two or more types of communicating components can be combined in the smart card readers **703** and **705-706** or incorporate another type of communicating component known to a person skilled in the art.

FIG. 8 illustrates another non-limiting embodiment of a smart card reader **804** as a part of a computer **805.** The computer **805** can contain at least the smart card reader **804,** the computer processor **801,** and the computer memory **802.** Optionally, the smart card reader **804,** the computer processor **801,** and the computer memory **802** can be detached and re-attached to the computer **805.** Alternatively, the listed parts cannot be readily detached from the computer **805.** The smart card reader **804** can communicate with a smart card (not shown) to relay personal health information stored in the smart card to the computer memory **802.** The personal health information can also be displayed on a screen **807** of a computer monitor **800,** which is connected to the computer **805** by a connector **806.** The computer **805** can have any suitable input component or device to allow a user to modify or update the personal health information and/or parameters for performing a therapy displayed on the screen **807,** provided that the smart card reader **804** has gained access to the smart card containing the personal health information or data. Optionally, the smart card reader **804** can contain any one or more of the communicating components illustrated in FIG.'s 7A- 7C. The computer processor **801** can contain an algorithm to generate a second password to be sent and stored in the smart card, substituting the function of the microprocessor of the smart card reader **804** as described in FIG. 4.

The smart card reader **804** can also be formed as a part of a medical system. The smart card reader **804** can further be connected as a part of the computer **805.** In addition to the possible functions of the smart card reader **804** as a part of the computer **805,** the smart card reader **804,** if attached to the medical system can read device configuration information stored in the smart card and send the information to the medical system to modify the function and/or the parameters of medical devices in the system.

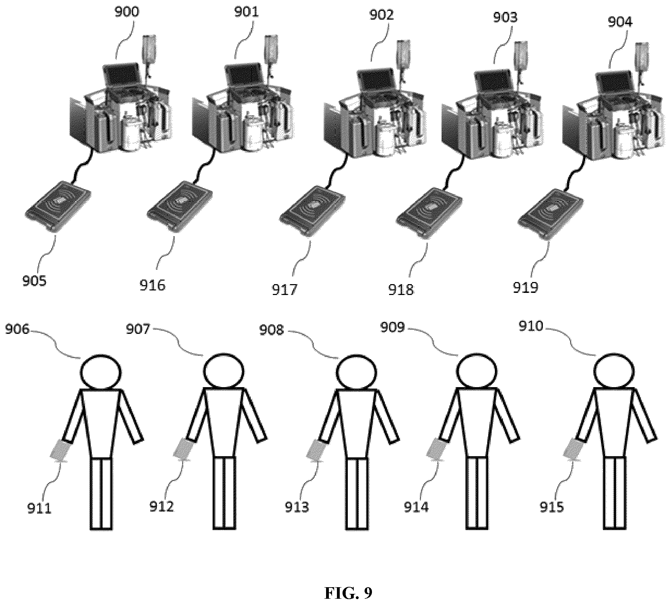

FIG. 9 illustrates a non-limiting embodiment of patients **906 - 910** with personal smart cards **911** - **915** accessing various medical systems **900, 901, 902, 903,** and **904** attached with smart card readers **905** and **916-919.** Each smart card **911, 912, 913, 914,** and **915** can contain protected personal health information or data necessary for performing therapy for each patient **906, 907, 908, 909,** and **910,** respectively. Optionally, the medical systems **900, 901, 902, 903,** and **904** can be dialysis machines or other medical systems that need to control access to data stored in smart cards **911, 912, 913, 914,** and **915.** In certain embodiments, smart card readers **905** and **916-919** can be incorporated into the medical systems **900, 901, 902, 903,** and **904** as a part of the systems, as described in FIG. 8.

Patients **906, 907, 908, 909,** and **910** holding their smart cards **911, 912, 913, 914,** and **915,** respectively, can individually access the medical systems **900, 901, 902, 903,** and **904** without the systems or the smart card readers **905** and **916-919** saving passwords for each smart card **911, 912, 913, 914,** and **915.** Instead, as explained in FIG. 4A and 4B, smart cards **911, 912, 913, 914,** and **915** can store their own second passwords. For example, a patient **906** with his/her smart card **911** can access any of the medical systems **900, 901, 902, 903,** and **904,** yet the medical systems **900, 901, 902, 903,** and **904** or the smart card readers **905** and **916-919** would not store the second password required to access the smart card **911** after the patient **906** is finished using any one of the medical systems **900, 901, 902, 903,** and **904.** Instead, the smart card readers **905** and **916-919** can generate the second password based at least partly on inputs such as a unique ID per the described algorithm. The smart card readers **905** and **916-919** generate an identical second password for each smart card **911, 912, 913, 914,** and **915,** regardless of which medical systems **900, 901, 902, 903,** and **904** the smart card readers **905** and **916-919** are attached. Alternatively, the smart card reader **905** and **916-919** can be configured to generate different second passwords depending on location or the type of the medical systems **900, 901, 902, 903,** and **904** to which the smart card readers **905** and **916-919** is attached. The generation of the second password can be processed by the medical systems **900, 901, 902, 903,** and **904,** not by the smart card readers **905** and **916-919.**

Alternatively, both the medical systems **900, 901, 902, 903,** and **904** and the smart card readers **905** and **916-919** can generate the second password. In any embodiment, the generated second password is not saved or stored in the medical systems **900, 901, 902, 903,** and **904** or the smart card readers **905** and **916-919** after the second password is generated and sent to the smart card **911, 912, 913, 914,** and **915** for verification.

In certain embodiments, even if one of the smart cards **915** is compromised and data is obtained by hackers, the hackers will not have access to other uncompromised smart cards **911, 912, 913,** and **914,** because each smart card **911, 912, 913, 914,** and **915** contains a unique second password. If the hackers infiltrate the smart card readers **905** and **916-919** or the medical systems **900, 901, 902, 903,** and **904,** they would not be able to acquire protected personal health information stored or other data in the smart cards **911, 912, 913, 914,** and **915** or the second passwords required to access them because the second passwords are not stored on the smart card readers **905** and **916-919** or the medical systems **900, 901, 902, 903,** and **904.**

In any of the described examples, the described systems, methods, and devices may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. As such, the term "processor" as used herein may refer to any of the foregoing structure or any other physical structure suitable for implementation of the described techniques. Also, the techniques could be fully implemented in one or more circuits or logic elements.

One skilled in the art will understand that various combinations and/or modifications and variations can be made in the described systems and methods depending upon the specific needs for operation. Various aspects disclosed herein may be combined in different combinations than the combinations specifically presented in the description and accompanying drawings. Moreover, features illustrated or described as being part of an aspect of the disclosure may be used in the aspect of the disclosure, either alone or in combination, or follow a preferred arrangement of one or more of the described elements. Depending on the example, certain acts or events of any of the processes or methods described herein may be performed in a different sequence, may be added, merged, or left out altogether (e.g., certain described acts or events may not be necessary to carry out the techniques). In addition, while certain aspects of this disclosure are described as performed by a single module or unit for purposes of clarity, the techniques of this disclosure may be performed by a combination of units or modules associated with, for example, a medical device.

The invention is described by the following numbered paragraphs: -
1. A secure medical system (100) for transmitting personal health information, comprising:
   a smart card (101);
   a smart card reader (102); and
   a microprocessor (503);
   wherein the smart card (101) has a secure computer readable memory having memory
   (501) allocated to store a first password verifiable by the smart card reader (102) and a second password (302) generated by the smart card reader; and a file directory (300) containing personal health information and a unique ID (200);
   wherein the smart card reader uses an algorithm (203) based at least partly on the unique ID to generate the second password; and
   the microprocessor comprising instructions for verifying a stored second password against a received second password sent by the smart card reader (402), the microprocessor providing access to the file directory containing data (502), if the stored second password matches the received second password (414).
2. The secure medical system of paragraph 1, the smart card further comprising an antenna (603), a capacitor (507), and a non-volatile memory unit (504).
3. The secure medical system of paragraph 2, wherein the antenna communicates with the smart card reader by transmitting and/or receiving radio frequency or wireless signals based on instructions from the microprocessor.
4. The secure medical system of any of paragraphs 1-3, the smart card further comprising a subscriber identification module chip (508), wherein the subscriber identification module chip makes direct electrical contact with the smart card reader (703) to transmit data.
5. The secure medical system of any of paragraphs 1-4, wherein the first password is a factory-preset password (400).
6. The secure medical system of any of paragraphs 1-5, wherein the unique ID is any one of a serial number, a globally unique identifier, or a hashed number (204).
7. The secure medical system of any of paragraphs 1-6, wherein the personal health information (502) comprises any one or more of a name, age, gender, height, weight, patient ID, prescription data, treatment data, and device configuration.
8. The secure medical system of any of paragraphs 1-7, wherein the first password is overwritten by the second password.
9. A smart card reader for use in a medical system, comprising:
   a microprocessor (600) having instructions for transmitting and receiving radio frequency or wireless signals and an algorithm for generating a second password;
   an antenna (603) for communicating with a smart card wherein the antenna transmits and/or receives radio frequency or wireless signals based on instructions from the microprocessor;
   a secure computer readable memory (601) storing a first password verifiable by the smart card and memory allocated for a unique ID received from the smart card;
   wherein the microprocessor further comprises instructions for generating the second password based at least partly on the unique ID received from the smart card, instructions for either transmitting and storing the second password on the smart card for initial preparation of the smart card, instructions for transmitting the second password to access a secured file directory containing data on the smart card, and instructions for discarding the second password.
10. The smart card reader of paragraph 9, wherein the algorithm is at least partly based on a partial secret (202) and number of iterations (201) of a pseudo-random function.
11. The smart card reader of paragraph 10, wherein a number of iterations of pseudo-random function is greater than 1.
12. The smart card reader of any of paragraphs 10-11, wherein the partial secret is identical across medical devices.
13. The smart card reader of any of paragraphs 10-12, wherein the pseudo-random function is HMAC-SHA1, HMAC-SHA2, HMAC-SHA3, or PBKDF2.
14. The smart card reader of any of paragraphs 9-13, wherein a length of the second password is at least 4 bytes.
15. The smart card reader of paragraph 9, further comprising a slot (701) for receiving the smart card, wherein the smart card reader transmits data to and/or from the smart card by direct electrical contact.
16. The smart card reader of any of paragraphs 9-15, wherein the smart card reader is in electrical communication with a desktop computer, a laptop computer, or any other medical system (103).
17. An automated smart card authentication system for use in a medical system, comprising:
   a smart card having memory allocated for storing a first password, a second password generated by an algorithm based on a unique ID, the unique ID, and personal health information;
   a smart card reader programmed to receive the unique ID and the first password (401) stored in the smart card (404); wherein the smart card reader generates a second password using the algorithm based at least partly on the unique ID of the smart card (406); the smart card reader storing the second password on the smart card (407), and discarding the second password from the smart card reader (408); wherein the smart card reader is programmed to generate the second password on each read of the smart card using the algorithm (410);
   wherein the smart card grants access to data if the second password generated by the smart card reader matches the second password stored in the smart card (412); and wherein the smart card reader discards the second password (415).
18. The automated smart card authentication system of paragraph 17, wherein the smart card reader transmits the data from the smart card to other medical devices connected to the smart card reader.
19. The automated smart card authentication system of any of paragraphs 17-18, wherein the second password generated by the smart card reader is stored on the smart card.
20. A method for automated smart card authentication for use in a medical system, comprising step of:
   accessing a smart card using a first password and a unique ID stored on the smart card (409), wherein the unique ID is used to generate a second password by a smart card reader (410), wherein the smart card reader uses an algorithm based at least partly on the unique ID to generate the second password wherein the second password is stored on the smart card and is discarded (415) and not stored by the smart card reader.
21. The method for automated smart card authentication for use in a medical system of paragraph 20, further comprising the step of:
   re-generating the same second password by the smart card reader using the unique ID of the smart card and upon verification of the same second password on the smart card gain access a file directory containing personal health information on the smart card (412).
22. A smart card for use in a medical system, comprising:
   a secure computer readable memory having a secure computer readable memory (501) having memory allocated to store a first password verifiable by a smart card reader (102) and a second password (302) generated by the smart card reader; a file directory containing personal health information, and a unique ID, wherein the smart card reader uses an algorithm based at least partly on the unique ID to generate the second password; and
   a microprocessor comprising instructions for verifying a stored second password against a received second password sent by the smart card reader, the microprocessor providing access to the file directory containing data, if the stored second password matches the received second password.
23. The smart card of paragraph 21, further comprising an antenna, a capacitor, and a non-volatile memory unit.
24. The smart card of paragraph 21, further comprising a subscriber identification module chip, wherein the subscriber identification module chip makes direct electrical contact with the smart card reader to transmit data.
25. The smart card of any of paragraphs 21-23, wherein the first password is overwritten by the second password.
26. The smart card of any of paragraphs 21-24, wherein the personal health information comprises any one or more of a name, age, gender, height, weight, patient ID, prescription data, treatment data, and device configuration.

## Claims

1. A secure medical system (100) for transmitting personal health information, comprising:
a smart card (101);
a smart card reader (102); and
a microprocessor (503);
wherein the smart card (101) has a secure computer readable memory having memory (501) allocated to store a first password verifiable by the smart card reader (102) and a second password (302) generated by the smart card reader; and a file directory (300) containing personal health information and a unique ID (200);
wherein the smart card reader uses an algorithm (203) based at least partly on the unique ID to generate the second password; and
the microprocessor comprising instructions for verifying a stored second password against a received second password sent by the smart card reader (402), the microprocessor providing access to the file directory containing data (502), if the stored second password matches the received second password (414).

2. The secure medical system of claim 1, the smart card further comprising an antenna (603), a capacitor (507), and a non-volatile memory unit (504), and preferably wherein the antenna communicates with the smart card reader by transmitting and/or receiving radio frequency or wireless signals based on instructions from the microprocessor.

3. The secure medical system of claim 1 or 2, the smart card further comprising a subscriber identification module chip (508), wherein the subscriber identification module chip makes direct electrical contact with the smart card reader (703) to transmit data.

4. The secure medical system of any of claims 1-3, wherein the first password is a factory-preset password (400).

5. The secure medical system of any of claims 1-4, wherein the unique ID is any one of a serial number, a globally unique identifier, or a hashed number (204).

6. A smart card reader for use in a secure medical system, comprising:
a microprocessor (600) having instructions for transmitting and receiving radio frequency or wireless signals and an algorithm for generating a second password;
an antenna (603) for communicating with a smart card wherein the antenna transmits and/or receives radio frequency or wireless signals based on instructions from the microprocessor; and
a secure computer readable memory (601) storing a first password verifiable by the smart card and memory allocated for a unique ID received from the smart card;
wherein the microprocessor further comprises instructions for generating the second password based at least partly on the unique ID received from the smart card, instructions for either transmitting and storing the second password on the smart card for initial preparation of the smart card, instructions for transmitting the second password to access a secured file directory containing data on the smart card, and instructions for discarding the second password.

7. The smart card reader of claim 6, wherein the algorithm is at least partly based on a partial secret (202) and number of iterations (201) of a pseudo-random function, preferably wherein the pseudo-random function is HMAC-SHA1, HMAC-SHA2, HMAC-SHA3, or PBKDF2.

8. The smart card reader of claim 7, wherein a number of iterations of the pseudo-random function is greater than 1.

9. The smart card reader of claim 7 or 8, wherein the partial secret is identical across medical devices and/or wherein a length of the second password is at least 4 bytes.

10. The smart card reader of any of claims 6 to 9, further comprising a slot (701) for receiving the smart card, wherein the smart card reader transmits data to and/or from the smart card by direct electrical contact and/or wherein the smart card reader is in electrical communication with a desktop computer, a laptop computer, or any other medical system (103).

11. An automated smart card authentication system for use in a medical system, comprising:
a smart card having memory allocated for storing a first password, a second password generated by an algorithm based on a unique ID, the unique ID, and personal health information;
a smart card reader programmed to receive the unique ID and the first password (401) stored in the smart card (404); wherein the smart card reader generates a second password using the algorithm based at least partly on the unique ID of the smart card (406); the smart card reader storing the second password on the smart card (407), and discarding the second password from the smart card reader (408); wherein the smart card reader is programmed to generate the second password on each read of the smart card using the algorithm (410);
wherein the smart card grants access to data if the second password generated by the smart card reader matches the second password stored in the smart card (412);
wherein the smart card reader discards the second password (415);
and optionally wherein: the smart card reader transmits the data from the smart card to other medical devices connected to the smart card reader and/or the second password generated by the smart card reader is stored on the smart card.

12. A smart card for use in a secure medical system, comprising:
a secure computer readable memory having a secure computer readable memory (501) having memory allocated to store a first password verifiable by a smart card reader (102) and a second password (302) generated by the smart card reader; a file directory containing personal health information, and a unique ID, wherein the smart card reader uses an algorithm based at least partly on the unique ID to generate the second password; and
a microprocessor comprising instructions for verifying a stored second password against a received second password sent by the smart card reader, the microprocessor providing access to the file directory containing data, if the stored second password matches the received second password;
and optionally further comprising a subscriber identification module chip, wherein the subscriber identification module chip makes direct electrical contact with the smart card reader to transmit data.

13. The smart card of claim 12 or the secure medical system of any of claims 1-5, wherein the first password is overwritten by the second password.

14. The smart card of claim 12 or 13 or the secure medical system of any of claims 1-5, wherein the personal health information comprises any one or more of a name, age, gender, height, weight, patient ID, prescription data, treatment data, and device configuration.

15. A method for automated smart card authentication for use in a medical system, comprising step of:
accessing a smart card using a first password and a unique ID stored on the smart card (409), wherein the unique ID is used to generate a second password by a smart card reader (410), wherein the smart card reader uses an algorithm based at least partly on the unique ID to generate the second password wherein the second password is stored on the smart card and is discarded (415) and not stored by the smart card reader;
and optionally further comprising the step of: re-generating the same second password by the smart card reader using the unique ID of the smart card and upon verification of the same second password on the smart card gain access a file directory containing personal health information on the smart card (412).
